# EUROPÄISCHE PATENTSCHRIFT

(11) **EP 2 278 875 B1**
(45) Veröffentlichungstag und Bekanntmachung des Hinweises auf die Patenterteilung: **05.11.2014**
(21) Anmeldenummer: 09727788.3
(22) Anmeldetag: 03.04.2009
(51) Int. Cl.: A01N 25/00, A01N 25/08, A01N 25/10, A01N 25/34, A01N 59/16, A01N 59/20, A61L 15/00, A61L 29/00

(54) **SCHICHTMATERIAL**
COATING MATERIAL
MATÉRIAU STRATIFIÉ

(30) Priorität: 04.04.2008 DE 102008001014
(43) Veröffentlichungstag der Anmeldung: 02.02.2011
(73) Patentinhaber: Bio-Gate AG, 28359 Bremen (DE); Fraunhofer-Gesellschaft zur Förderung der angewandten Forschung e.V., 80686 München (DE)
(72) Erfinder: WAGENER, Michael, 28355 Bremen (DE); SALZ, Dirk, 28195 Bremen (DE); VISSING, Klaus-Dieter, 27321 Morsum (DE)
(74) Vertreter: Eisenführ Speiser
(86) Internationale Anmeldenummer: PCT/EP2009/054049
(87) Internationale Veröffentlichungsnummer: WO 2009/121970

(56) Entgegenhaltungen:
- WO-A1-2008/068948
- WO-A2-2005/049699

## Beschreibung

Die Erfindung betrifft ein antimikrobielles und nicht zytotoxisches Schichtmaterial und Verwendungen dieses Schichtmaterials.

Es besteht ein unablässiger Bedarf in vielfältigen Anwendungsgebieten, die Ansiedlung, die Vermehrung und das Überleben von Mikroorganismen, insbesondere Prokaryonten und Pilzen, zu steuern. Insbesondere ist es vielfach gewünscht, die Konzentration von Mikroorganismen auf einer bestimmten Fläche zu begrenzen oder diese Fläche gänzlich von Mikroorganismen - gegebenenfalls Mikroorganismen einer bestimmten Art oder Gattung - freizuhalten. Dieses Ziel wird insbesondere in im weitesten Sinne medizinischen, medizintechnischen oder hygienetechnischen Anwendungen angestrebt. Herkömmlicherweise werden deshalb beispielsweise im Bereich von Medizin- und Hygieneprodukten antimikrobiell wirksame Werkstoffe und Beschichtungen verwendet, beispielsweise silberbeschichtete Fäden für die Chirurgie (siehe S. Silver, FEMS Microbiology Reviews (2003): 341 bis 353) oder kupferhaltige Antifoulinglacke. Als besonders wirksam haben sich dabei breitbandig wirksame Biozide und hierbei insbesondere anorganische Biozide wie beispielsweise Silber und dessen Ionen erwiesen. Das mit dem Biozid behandelte Material setzt dabei im Laufe der Zeit das in ihm enthaltene Biozid frei und verringert oder verhindert vollständig die Ansiedlung oder Vermehrung von Mikroorganismen auf dem Material selbst, aber auch in seiner Umgebung.

Dabei ist häufig problematisch, dass die herkömmlichen antimikrobiell wirksamen Materialien anfänglich eine hohe Biozid-Konzentration freisetzen, so dass die Konzentration des freigesetzten Biozids nicht nur auf die zu bekämpfenden Mikroorganismen, sondern ungewollt auch auf höhere Zellen toxisch wirkt. Dies ist insbesondere bei Medizinprodukten wie Wundauflagen, Kathetern, Kontaktlinsen und Implantaten störend, da ein so behandeltes Medizinprodukt die Wundheilung verzögern und Gewebereizungen und Allergien hervorrufen kann. Entsprechende Nachteile treten auch bei Biozid-freisetzenden Hygieneprodukten wie beispielsweise Binden, Tampons oder Windeln sowie bei der Herstellung und Verarbeitung von Lebensmitteln auf, insbesondere im Zusammenhang mit Biozid-freisetzenden Verpackungen sowie Biozid-freisetzenden Bauteilen zum Herstellen oder Verarbeiten von Lebensmitteln. Darüber hinaus wird die antimikrobielle Wirkung durch Auslaugung des mit dem bioziden Wirkstoff versehenen Materials rasch erschöpft.

Zum Beheben dieser Nachteile wird gemäß der WO 03/024494 ein antimikrobieller Kleb- und Beschichtungsstoff vorgeschlagen, der metallische Silber-Partikel mit einem Gehalt von weniger als 5 ppm an Silber-, Natrium- und Kalium-lonen enthält, wobei es sich bei dem Kleb- und Beschichtungsstoff um ein synthetisch hergestelltes Material auf organischer Basis handelt, das im Allgemeinen nach der Verarbeitung aushärtet. Die Silber-Partikel sind dabei gleichmäßig im Kleb- und Beschichtungsstoff verteilt. Insbesondere soll der Kleb- und Beschichtungsstoff ein Lack oder Klebstoff insbesondere auf duro- oder thermoplastischer Basis sein. Nachteilig hieran ist jedoch, dass die Metallionenfreisetzungsrate nur schwer steuer- oder einstellbar ist.

Es war deshalb eine Aufgabe der vorliegenden Erfindung, ein möglichst einfach und kostengünstig herstellbares Schichtmaterial anzugeben, das antimikrobielle Eigenschaften besitzt, jedoch nicht zytotoxisch sein sollte. Dabei ist ein Schichtmaterial antimikrobiell, wenn es die Vermehrung von *Staphylococcus epidermidis* zumindest zehn Stunden lang hemmt, gemessen wie beschrieben in DE 197 58 598 A1. Ein Schichtmaterial ist ferner zytotoxisch, wenn es eine zytotoxische Wirkung wie in DIN-ISO 10993-5 beschrieben aufweist. Das Schichtmaterial sollte zudem eine möglichst langanhaltende antimikrobielle und nicht zytotoxische Wirkung besitzen. Es sollte möglichst universell einsetzbar sein und die Herstellung auch dünner Beschichtungen ermöglichen.

Erfindungsgemäß wird deshalb ein antimikrobielles und nicht zytotoxisches Schichtmaterial vorgeschlagen, umfassend
a) eine Trägerschicht,
b) zumindest eine auf der Trägerschicht aufgebrachte Biozid-Schicht mit einem anorganischen bioziden Wirkstoff, ausgewählt aus der Gruppe bestehend aus Silber, Kupfer und Zink, deren Ionen und deren Metallkomplexe, oder einer Mischung oder Legierung umfassend zwei oder mehr dieser Elemente, und
c) eine auf eine Biozid-Schicht aufgebrachte Transportkontrollschicht, wobei die Transportkontrollschicht eine Gasdurchlässigkeit für Sauerstoff (O₂) aufweist von 50 bis unter 100 (cm³ bar) / (Tag m²), wobei die Trägerschicht und die Transportkontrollschicht das gleiche Grundmaterial besitzen.

Mit der erfindungsgemäß gewählten Gasdurchlässigkeit der Transportkontrollschicht für Sauerstoff ist es möglich, den bioziden Wirkstoff aus der Biozid-Schicht durch die Transportkontrollschicht hindurch in einer antimikrobiell wirksamen und gleichzeitig nicht zytotoxischen Menge abzugeben. Gegenüber herkömmlichen antimikrobiellen Materialien ermöglicht es das erfindungsgemäße Schichtmaterial, eine hohe Biozid-Konzentration im Schichtmaterial selbst vorzusehen, die normalerweise zytotoxisch wirken würde. Die Biozid-Schicht bildet gleichsam ein Depot des bioziden Wirkstoffs, um eine langanhaltende Abgabe des bioziden Wirkstoffs zu ermöglichen. Durch Vorsehen der Transportkontrollschicht mit der oben angegebenen Gasdurchlässigkeit ist es darüber hinaus möglich, die Konzentration des aus der Biozid-Schicht durch die Transportkontrollschicht hindurch abgegebenen bioziden Wirkstoffs zu begrenzen, so dass dieser nicht mehr zytotoxisch, gleichzeitig jedoch auch noch immer antimikrobiell wirkt. Die Transportkontrollschicht hat somit eine steuernde und regulierende Funktion. Darüber hinaus kann die Transportkontrollschicht einen direkten Kontakt der Umgebung mit der Biozid-Schicht verhindern. Dadurch wird die Haltbarkeit des Schichtmaterials verbessert. Die Transportkontrollschicht kann auf beiden Seiten oder nur auf einer Seite der Biozid-Schicht angeordnet sein. Letzteres ist insbesondere dann bevorzugt, wenn das erfindungsgemäße Schichtmaterial eine Beschichtung auf einem festen Körper bildet. In einem solchen Fall kann der mit dem erfindungsgemäßen Schichtmaterial beschichtete Körper die nicht von der Transportkontrollschicht bedeckte Seite der Biozid-Schicht bedecken.

Im Sinne der vorliegenden Textes ist ein biozider Wirkstoff jede Substanz, die eine antimikrobielle Wirkung im oben beschriebenen Sinne entfalten kann (biozider Wirkstoff im engeren Sinne). Zu den bioziden Wirkstoffen werden auch solche Stoffe gezählt, die durch Umwandlung den bioziden Wirkstoff im engeren Sinne in der Umgebung hervorbringen, in der ein jeweiliges Schichtmaterial bestimmungsgemäß verwendet werden soll. Ist beispielsweise der biozide Wirkstoff im engeren Sinne ein Metallion, insbesondere ein Silber-, Kupfer- und/oder Zink-Kation, so sind auch metallisches Silber, Kupfer bzw. Zink und Legierungen, Komplexe und andere Substanzen biozide Wirkstoffe, aus denen die genannten Kationen in einem geeigneten Umfeld freigesetzt werden können, beispielsweise im Bereich einer Wunde.

Überraschenderweise hat sich nunmehr herausgestellt, dass insbesondere bei Verwendung von Silber als biozidem Wirkstoff durch die gewählte Sauerstoff-Gasdurchlässigkeit eine besonders lang antimikrobiell wirksame Beschichtung erreicht werden kann. Dies war um so überraschender, als zu erwarten war, dass Wasser, das zum Freisetzen von Silberionen aus partikelförmigem Silber benötigt wird, nicht in einem ausreichenden Maß die Transportkontrollschicht würde passieren können, so dass eine ausreichende antimikrobielle Wirkung nicht erwartet werden konnte.

Der Fachmann versteht, dass ein erfindungsgemäßes Schichtmaterial auch gegen andere Mikroorganismen und nicht oder nicht nur gegen *Staphylococcus epidemidis* antimikrobiell wirksam sein kann. Die antimikrobielle Wirksamkeit des erfindungsgemäßen Schichtmaterials im Hinblick auf andere Mikroorganismen wird entsprechend der DE 197 58 598 A1 mit dem jeweils zu untersuchenden Mikroorganismus anstelle von *Staphylococcus epidermidis* durchgeführt. Besonders bevorzugt sind solche erfindungsgemäßen Schichtmaterialien, die, ohne zytotoxisch zu sein, antimikrobiell wirksam sind gegen einen oder mehrere der Mikroorganismen der Gruppe *Bacillus, Clostridium, Enterobacter, Escherichia, Pseudomonas, Salmonella, Staphylococcus, Yersinia, Candida.*

Der biozide Wirkstoff kann zum Bilden einer Biozid-Schicht insbesondere in die Transportkontrollschicht und/oder die Trägerschicht eingebettet sein. In bevorzugten Ausführungsformen der Erfindung umfasst das Schichtmaterial daher einen partikelförmigen bioziden Wirkstoff, insbesondere partikelförmiges Silber, wobei die einzelnen Partikel in die Trägerschicht und/oder die Transportkontrollschicht eingebettet sind.

Der biozide Wirkstoff und insbesondere Silber als Bakterizid liegt vorzugsweise als gegebenenfalls teilweise miteinander verschmolzene Teilchen vor wie sie bei Vakuumverdampfen, Sputtern und chemischer Dampfabscheidung (chemical vapor deposition) erhältlich und unten weiter beschrieben sind. Dabei sind die Teilchen Vollmaterial-Teilchen und nicht mit dem bioziden Wirkstoff ummantelte Trägerteilchen.

Die Transportkontrollschicht des erfindungsgemäßen Schichtmaterials ist vorzugsweise so ausgebildet, dass die Transportkontrollschicht eine Gasdurchlässigkeit für Sauerstoff (O₂) aufweist von unter 100 (cm³ bar) / (Tag m²), vorzugsweise von 50 bis unter 100 (cm³ bar) / (Tag m²⁾). Solche Transportkontrollschichten können besonders zweckmäßig durch Plasmapolymerisation erzeugt werden. Anhand des soeben beschriebenen Gasdurchlässigkeitskriteriums kann der Fachmann unter Durchführung üblicher Routineversuche geeignete Ausgangsmaterialien und Parameter für die Herstellung einer entsprechenden Transportkontrollschicht ermitteln. Besonders bevorzugte Transportkontrollschichten sind im weiteren Verlauf dieser Beschreibung und in den Beispielen angegeben.

Die Transportkontrollschicht kann mit Hilfe der Vakuumtechnik hergestellt werden. Insbesondere bei verdampfbaren, organischen Verbindungen ist die Methode der Plasmapolymerisation (PE-CVD) geeignet, eine Transportkontrollschicht auf einem Substrat abzuscheiden. Organische Verbindungen sind z.B. gesättigte oder ungesättigte, lineare oder cyclische Kohlenwasserstoffe, die gegebenenfalls zusätzliche Funktionalitäten (z.B. Substituenten) aufweisen können. Diese optionalen Funktionalitäten sind vorzugsweise heteroatomhaltig, besonders bevorzugt sind Hydroxyl-, Carboxylgruppen oder Amine. Neben den organischen Verbindungen können auch metallorganische Verbindungen zum Herstellen einer Transportkontrollschicht durch Plasmapolymerisation eingesetzt werden. In soweit bevorzugt ist insbesondere die Abscheidung von Titanoxid und Vanadiumoxid; die Transportkontrollschicht wird in diesen Fällen besonders bevorzugt hergestellt durch die plasmagestützte Polymerisation von Titanisopropoxid bzw. Vanadiumtriisopropoxid. Wird der Dampfdruck der metallorganischen Verbindung durch Erwärmen erhöht, so lassen sich auch Wolframoxide aus Wolframethoxid und Zinnoxid aus dem ungiftigen Tetravinylzinn herstellen.

Die Abscheidung der Transportkontrollschicht kann weiterhin durch Sputtern erfolgen, wobei eine Gleich- (DC), Mittelfrequenz- (MF) und Hochfrequenz- (HF) -Spannung verwendet werden kann. Zur Abscheidung oxidischer Schichten (z.B. Titanoxid, Hafniumoxid) wird das metallische Target im reaktiven DC-Sputtermodus in einer Ar/02-Atmospäre betrieben. Alternativ kann aber auch im Hochfrequenzmodus gearbeitet werden. Neben den Oxiden können prinzipiell auch Karbide, Sulfide, Titanate, Vanadate, Wolframate, Selenate und Molybdate verwendet werden. Hierbei wird ein Kathodenmaterial verwendet, das dem Beschichtungsmaterial entspricht. Da die oben aufgeführten Verbindungen Isolatoren sind, können solche Beschichtungen mit einer Hochfrequenzspannung abgesputtert werden. Im Allgemeinen ergibt sich aber kein Schichtmaterial, welches der chemischen Zusammensetzung des Targetmaterials entspricht. Abweichungen von der Stöchiometrie sind bei diesem Verfahrensansatz üblich.

Neben den antibakteriellen, nicht zytotoxischen Eigenschaften können weitere Schichtfunktionen eine wichtige Rolle spielen. Im Falle von Implantaten, die eine hohe mechanische Verschleißfestigkeit aufweisen müssen, ist eine hohe Schichthärte erforderlich und durch die erfindungsgemäße Transportkontrollschicht erreichbar. Vorzugsweise besitzt das erfindungsgemäße Schichtmaterial und insbesondere die Transportkontrollschicht eine Härte von größer als 1 GPa bei Nanoindentationsmessung.

Ferner ist es bevorzugt, wenn die Oberflächenenergie, die die Zelladhäsion beeinflusst, von 40 bis 110 mN/m beträgt. Besonders bevorzugt im Falle langzeitstabiler Oberflächen sind Oberflächenenergien von 50 - 110 mN/m. Für schmutzabweisende Schichtmaterialien beträgt die Oberflächenenergie weniger als 30 mN/m. Diese Oberflächeneigenschaft kann gegebenenfalls durch eine sehr dünne zusätzliche Beschichtung eingestellt werden.

Die Einsatzdauer der Beschichtung bzw. der Zeitraum, in dem das Bakterizid freigegeben werden soll, kann durch den Aufbau von mehreren unmittelbar aufeinander folgenden Biozid- und Transportkontrollschichten (Multilayer) verlängert werden. Bevorzugt ist in soweit insbesondere eine Beschichtung mit dem Aufbau Ag/SiO₂/Ag/SiO₂/Ag/SiO₂/Ag/SiO₂, diese weist im Vergleich zur Beschichtung Ag/SiO₂ eine deutlich längere antibakterielle Wirkung auf. Weiterhin besteht die Möglichkeit, durch Wahl des Schichtaufbaus die Farbe der Beschichtung einzustellen. Werden zwei Ag/SiO₂-Schichten übereinander abgeschieden und die Farbe der ersten Beschichtung ist gelb und die der zweiten ist blau, so entsteht als resultierende Farbe der gesamten Beschichtung grün.

Bei vielen, speziell medizinischen Anwendungen, ist die Sterilisierbarkeit des beschichteten Substrats eine wichtige Grundvoraussetzung für die Einsatzfähigkeit. Der Schichtaufbau bzw. die Verpackung muss dann so gewählt sein, dass die Beschichtung durch Gammastrahlen und Autoklavieren bzw. durch die Behandlung mit Ethylenoxid nicht zum Versagen der Beschichtung führt. Schichten, die sich autoklavieren bzw. mit Ethylenoxid sterilisieren lassen, müssen eine gute Haftung zum Substrat aufweisen. Eine Substratvorbehandlung im Sauerstoffplasma ist deshalb für diesen Anwendungsbereich besonders bevorzugt.

Weiterhin ist zu beachten, dass für eine Autoklavierbarkeit die Beschichtung temperaturbeständig bis mindestens 200°C sein sollte. Diese Temperaturbeständigkeit ist bei praktisch allen Oxiden vorhanden, sowie bei vielen hochvernetzten rein organischen Schichten. Die in dieser Beschreibung und den Beispielen dargestellten Herstellverfahren für Transportkontrollschichten und deren Materialien kann der Fachmann in Kenntnis dieser Erfindung gegebenenfalls unter Zuhilfenahme seines allgemeinen Fachwissens ergänzen, um ohne Weiteres durch Routinearbeiten Transportkontrollschichten mit anderen Materialien und/oder Herstellverfahren zu entwickeln.

Anorganische biozide Wirkstoffe sind gewöhnlich preiswert, leicht erhältlich und leicht zu verarbeiten. Der biozide Wirkstoff kann durch verschiedene Verfahren vorgelegt werden, insbesondere kann er auf einer Oberfläche aufgebracht werden, die mit einem erfindungsgemäßen Schichtmaterial beschichtet werden soll. Zum Aufbringen eines anorganischen bioziden Wirkstoffs besonders geeignet sind Vakuumverdampfen, Sputtern und chemical vapor deposition.

Die erfindungsgemäß einzusetzenden bioziden Wirkstoffe wirken gegen eine Vielzahl verschiedener Mikroorganismen und greifen auf zahlreiche Weisen in deren Stoffwechsel ein. Dementsprechend kommt es bei Verwendung dieser bioziden Wirkstoffe seltener zur Resistenzbildung bei Bakterien als bei Verwendung spezifisch wirkender organischer Biozide, insbesondere Antibiotika.

Als besonders vorteilhaft hat sich dabei ein solches erfindungsgemäßes Schichtmaterial herausgestellt, bei dem der biozide Wirkstoff Silber, ein Silber-Kation oder ein Silber- bzw. Silberkation-freisetzender Komplex oder eine solche Legierung ist. Insbesondere metallisches Silber ist leicht verarbeitbar und in hoher Qualität zu einem verhältnismäßig geringen Preis erhältlich, so dass auch das erfindungsgemäße Schichtmaterial wiederum verhältnismäßig preiswert hergestellt werden kann.

Zweckmäßigerweise liegt der biozide Wirkstoff im erfindungsgemäßen Schichtmaterial in körniger Form vor, wobei eine mittlere Korngröße der Primärpartikel von 5 bis 100 nm bevorzugt ist. Solche feinen Pulver biozider Wirkstoffe lassen sich insbesondere für anorganische Biozide, und hierbei insbesondere für Silber, aber auch für Kupfer und Zink, sowie Mischungen, Komplexe und Legierungen der drei genannten Metalle leicht herstellen. Aufgrund der geringen mittleren Korngröße besitzt der biozide Wirkstoff eine hohe spezifische Oberfläche, so dass er insbesondere durch Diffusion gut aus der Biozid-Schicht heraus abgegeben werden kann. Ferner ist vorteilhaft, dass aufgrund der hohen spezifischen Oberfläche eine chemische Inaktivierung des körnigen Wirkstoffs, wie sie beispielsweise in Wundumgebungen gelegentlich zu besorgen ist, gewöhnlich nur einen Teil der Oberfläche betrifft, so dass eine Abgabe des bioziden Wirkstoff aus der Biozid-Schicht heraus auch unter widrigen Bedingen ermöglicht wird. Als besonders vorteilhaft haben sich solche erfindungsgemäßen Schichtmaterialien erwiesen, bei denen die mittlere Korngröße des bioziden Wirkstoffs 5 bis 50 nm, vorzugsweise 5 bis 20 nm betrug. Wenn der biozide Wirkstoff Silber oder eine Silber-Legierung ist, so spricht man bei diesen Korngrößenverteilungen auch von nanoskaligem (nano scale) Silber bzw. einer nanoskaligen Silber-Legierung.

Bei Verwendung von Silber, Kupfer und Zink, deren Ionen und deren Metallkomplexe oder einer Mischung oder Legierung dieser Elemente als biozidem Wirkstoff ist es besonders bevorzugt, wenn der biozide Wirkstoff keinen unmittelbaren Kontakt mit einem elektrisch leitfähigem Substrat besitzt. Es ist deshalb insbesondere in dem Fall, dass die erfindungsgemäße Beschichtung auf einem Metall-Substrat, wie beispielsweise einem Edelstahl, Titan oder Titanlegierungen aufgebracht werden soll, bevorzugt, durch die Trägerschicht eine elektrisch isolierende Trennung zwischen dem bioziden Wirkstoff und dem Substrat zu bewirken.

Die Biozid-Schicht kann, je nach Anwendungsbereich, eine Dicke von zumindest 1 nm und vorzugsweise zumindest 3 nm, und ferner vorzugsweise nicht mehr als 1 mm und vorzugsweise nicht mehr als 100 nm besitzen. Umfasst das Schichtmaterial mehrere Biozid-Schichten, so gelten diese Dicken für jede einzelne Biozid-Schicht. Die Biozid-Schicht braucht - abgesehen von der Anwesenheit des bioziden Wirkstoffs - nicht aus einem anderen Material zu bestehen als die Trägerschicht und/oder die Transportschicht, vielmehr kann der biozide Wirkstoff auch in eine oder zwischen beide Schichten eingebettet sein. Dann ist mit "Dicke der Biozid-Schicht" die Dicke desjenigen Bereichs der Trägerschicht und/oder der Transportkontrollschicht gemeint, in dem der biozide Wirkstoff eingebettet ist. Bei Verwendung körniger biozider Wirkstoffe ist die Biozid-Schicht daher zumindest so dick wie der körnige Wirkstoff. Vorzugsweise beträgt die Dicke der Biozid-Schicht zumindest 1 nm bis 100 nm, wobei besonders Schichtdicken von 3 nm bis 50 nm bevorzugt werden, insbesondere wenn der biozide Wirkstoff Silber, Kupfer und/oder Zink bzw. deren Ionen, Metallkomplexe oder eine Mischung oder Legierung dieser Elemente ist. Es hat sich gezeigt, dass in einem erfindungsgemäßen Schichtmaterial bereits derartig geringe Schichtdicken eines bioziden Wirkstoffs (insbesondere eines bioziden Wirkstoffs enthaltend nanoskaliges Silber) ausreichend sind, um eine antimikrobielle, nicht cytotoxische Wirkung dauerhaft erreichen zu können.

Ferner ist ein erfindungsgemäßes Schichtmaterial bevorzugt, bei der die Biozid-Schicht ferner umfasst: Gold, Platin, Palladium, Iridium, Zinn, Antimon, deren Ionen, deren Metallkomplexe, oder eine Mischung oder Legierung des bioziden Wirkstoffs mit einem oder mehreren dieser Elemente. Der Zusatz der genannten Elemente zum bioziden Wirkstoff erhöht und/oder verlängert die antimikrobielle Wirksamkeit. Die genannten Elemente liegen vorzugsweise in kationischer Form gebunden in Ionenaustauschern, in Form eines Komplexes oder als Salz, vorzugsweise einer polymeren Carbonsäure, vor.

Darüber hinaus ist ein erfindungsgemäßes Schichtmaterial bevorzugt, bei dem die Transportkontrollschicht und/oder die Trägerschicht ein Grundmaterial besitzt, das ausgewählt ist aus der Gruppe bestehend aus
a) einem organischen Grundmaterial, insbesondere einem Plasmapolymer, einem Sol-Gel, einem Lack, und einem silikonisierten Grundmaterial, oder
b) einem anorganischen Grundmaterial, insbesondere SiO₂ und SiC, einem Metalloxid, insbesondere TiO₂ und Al₂O₃, und einem nicht-bioziden Metall, insbesondere Titan oder medizinischem Edelstahl.

Es versteht sich dabei, dass das Grundmaterial eine Dicke und Durchlässigkeit besitzt, um eine Abgabe des bioziden Wirkstoffs durch die Transportkontrollschicht hindurch in einer Konzentration zu ermöglichen, bei der der so abgegebene biozide Wirkstoff antimikrobiell und nicht cytotoxisch wirken kann. Hierbei ist besonders bevorzugt, wenn das Grundmaterial mikroporös ist. Insbesondere zum Herstellen dünner Schichten ist es bevorzugt, die Transportkontrollschicht durch Plasma-Polymerisationsverfahren oder durch Aufsputtern herzustellen. Auf diese Weise können sehr dünne Transportkontrollschichten hergestellt werden, durch die biozide Wirkstoffe, wie beispielsweise atomares oder kationisches Silber diffundieren und dem Schichtmaterial seine antimikrobielle, nicht cytotoxische Wirkung verleihen können.

Die Transportkontrollschicht wird vorzugsweise so hergestellt, dass ihre Schichtdicke, Dichte, ihr Feuchtigkeitsaufnahmevermögen, ihre Diffusionsdichtigkeit gegen Wasserdampf, ihre chemische Zusammensetzung und ihre Vernetzungsstruktur eine Abgabe des bioziden Wirkstoffs durch die Transportkontrollschicht hindurch ermöglicht, so dass der so abgegebene biozide Wirkstoff antimikrobiell und nicht cyclotoxisch wirken kann. Dient beispielsweise eine plasmapolymere Schicht als Transportkontrollschicht, so ist diese vorzugsweise stark vernetzt und besitzt eine niedrige spezifische Durchlässigkeit für Wasserdampf sowie ein geringes Feuchtigkeitsaufnahmevermögen. Eine derartige Transportkontrollschicht benötigt nur eine sehr geringe Schichtdicke, um noch eine ausreichende antimikrobielle, aber noch nicht cytotoxische Wirksamkeit des bioziden Wirkstoffs zu gewährleisten.

Besonders bevorzugt ist ein solches erfindungsgemäßes Schichtmaterial, bei dem die Trägerschicht und/oder Transportkontrollschicht einen Siliciumanteil von 20 bis 60 %, einen Kohlenstoffanteil von 10 bis 30 % und einen Sauerstoffanteil von 30 bis 50 % besitzt. Es versteht sich dabei, dass die Anteile so aufeinander abgestimmt sein müssen, dass sie insgesamt nicht mehr als 100 % ergeben. Die Anteile werden dabei durch X-ray Photoelectron-Spektroskopie (XPS) ermittelt; dabei bleiben Elemente bei der Bestimmung des Silicium-, Kohlenstoff- und Sauerstoffanteils außer Betracht, die beispielsweise wie Wasserstoff nicht durch XPS-Analyse bestimmt werden können. Es können also neben Silicium, Kohlenstoff und Sauerstoff noch weitere Elemente in der Trägerschicht und/oder Transportkontrollschicht vorhanden sein (nämlich solche, die durch XPS nicht nachgewiesen werden können), ohne dass diese weiteren Elemente bei der Bestimmung des Silicium-, Kohlenstoff- und Sauerstoffanteils berücksichtigt würden. Der Silicium-, Kohlenstoff- und Sauerstoffanteil wird in Atomprozent bzw. Molprozent der durch XPS-Analyse nachweisbaren Elemente angegeben.

Die Trägerschicht und/oder Transportkontrollschicht eines erfindungsgemäßen Schichtmaterials hat vorzugsweise eine mittlere Dicke von 5 nm bis 500 nm. Insbesondere bei Verwendung einer plasmapolymeren Trägerschicht und/oder plasmapolymeren Transportkontrollschicht ist es jedoch bevorzugt, wenn die Trägerschicht und/oder Transportkontrollschicht eine Dicke von 5 bis 200 nm, vorzugsweise 10 bis 100 nm besitzt. Bei diesen Schichtdicken lassen sich insbesondere mit durch Plasmapolymerisation hergestellte Trägerschichten und/oder Transportkontrollschichten hervorragende antimikrobielle und nicht zytotoxische Schichtmaterialien herstellen. Gleichzeitig sind diese Trägerschichten und/oder Transportkontrollschichten sehr dünn, so dass sie optisch kaum auffallen oder sogar transparent sein können.

Besonders vorteilhaft ist es, wenn die Biozid-Schicht und Trägerschicht und/oder Transportkontrollschicht ein allen gemeinsames Grundmaterial besitzen. Auf diese Weise ist es insbesondere möglich, zunächst einen bioziden Wirkstoff (insbesondere Silber, Kupfer und/oder Zink) in vorzugsweise nanoskaliger Form auf einer Trägerschicht vorzulegen und anschließend, soweit gewünscht, durch Auftragen des Grundmaterials einer weiteren Trägerschicht eine oder mehrere weitere Biozid-Schichten aufzubringen. Anschließend ist es möglich, durch Auftragen des Grundmaterials der Transportkontrollschicht in einem einzigen weiteren Arbeitsschritt das erfindungsgemäße Schichtmaterial herzustellen und dabei den bioziden Wirkstoff in diesem Schichtmaterial einzubetten.

Das Grundmaterial der Transportkontrollschicht kann ferner so ausgewählt werden, dass die Transportkontrollschicht außer der Eigenschaft, die Abgabe des bioziden Wirkstoffs durch die Transportkontrollschicht hindurch zu ermöglichen, weitere und vorteilhafte Eigenschaften besitzt. Insbesondere kann die Transportkontrollschicht durch geeignete Wahl des Grundmaterials oder durch weitere Maßnahmen transparent, hydrophil, hydrophob und/oder (auch für Bakterien) nicht-haftend sein.

Besonders bevorzugt besitzen die Trägerschicht und die Transportkontrollschicht das gleiche Grundmaterial.

Ferner ist erfindungsgemäß ein Schichtmaterial bevorzugt, das mehrere Trägerschichten und Biozidschichten umfasst, so dass zumindest eine Biozidschicht eingebettet ist zwischen zwei Trägerschichten. Auf diese Weise kann das Schichtmaterial einen höheren Gehalt an biozidem Wirkstoff umfassen, als durch Einbetten in einer einzigen Schicht erreichbar wäre. Insbesondere wenn der biozide Wirkstoff ein partikelförmiger Wirkstoff ist kann es sein, dass die Dicke der Biozidschicht eine vom Grundmaterial der Trägerschicht und/oder Transportkontrollschicht abhängige Maximaldicke nicht überschreiten darf, ohne dass das Schichtmaterial Einbußen in der mechanischen Stabilität und insbesondere der Abriebfestigkeit hätte. Durch Verwendung mehrerer Biozid-Schichten, die jeweils höchstens die Maximaldicke aufweisen, ist es möglich, den Gehalt an biozidem Wirkstoff im erfindungsgemäßen Schichtmaterial ohne nennenswerte Einbußen in der mechanischen Stabilität zu erhöhen.

Dabei war zu erwarten gewesen, dass durch die Vergrößerung der Dicke des erfindungsgemäßen Schichtmaterials und dementsprechend durch Vergrößerung des Abstandes zwischen der äußeren Oberfläche der Transportkontrollschicht und der von ihr am weitesten beabstandeten Biozid-Schicht die Diffusion des bioziden Wirkstoffs aus dieser Biozid-Schicht zur Oberfläche der Transportkontrollschicht zu stark behindert wird, als dass eine Freisetzung des bioziden Wirkstoffs noch erfolgen würde. Falls aber eine Diffusion möglich wäre, so war zu befürchten, dass durch das Erhöhen des Gehalts an biozidem Wirkstoff das Schichtmaterial den Wirkstoff in einer höheren und damit zytotoxischen Menge freisetzen wird. Es hat sich überraschend jedoch herausgestellt, dass der biozide Wirkstoff auch aus der am weitesten beabstandeten Biozid-Schicht noch an die Oberfläche der Transportkontrollschicht gelangt und dort antimikrobiell und nicht zytotoxisch wirkt.

Besonders bevorzugte Trägerschicht- und Transportschicht-Kombinationen sind: SiO₂ / Ag / SiO₂; TiO₂/Ag/TiO₂; organische Beschichtung/Ag/organische Beschichtung; Beschichtungssysteme mit Silber und Antireflexwirkung.

Die Biozid-Schicht und auch das erfindungsgemäße Schichtmaterial insgesamt können in beliebiger Form vorliegen. Insbesondere kann die Biozid-Schicht und das erfindungsgemäße Schichtmaterial eine Beschichtung auf einem festen Körper bilden, beispielsweise auf einer Faser, auf einer Metall-, Kunststoff- und/oder Glas-Oberfläche. Die Biozid-Schicht und das erfindungsgemäße Schichtmaterial können aber auch eine Beschichtung auf Partikeln bilden.

Bei Verwendung von Silber (insbesondere nanoskaligem Silber) als biozidem Wirkstoff beträgt der Silbergehalt des erfindungsgemäßen Schichtmaterials vorzugsweise 1 bis 100 ppm. Es sich überraschenderweise gezeigt, dass in einem erfindungsgemäßen Schichtmaterial festes Silber bereits in den angegebenen Mengen eine ausreichend antimikrobielle Wirkung entfalten kann.

Erfindungsgemäß ist das zuvor beschriebene Schichtmaterial einschließlich seiner Ausführungsformen verwendbar zum Herstellen einer antimikrobiellen und nicht zytotoxischen Beschichtung auf einem festen Körper. Insbesondere ist es verwendbar zum Herstellen einer antimikrobiellen und nicht zytotoxischen Beschichtung auf einem Medizinprodukt, insbesondere einem Katheter, einer Wundauflage, einer Kontaktlinse, einem Implantat, einem medizinischen Nagel und/oder Schraube, Knochenfixationsnagel, einem medizinischen Instrument, oder auf einem Hygieneprodukt, insbesondere einer Binde, einem Tampon oder einer Windel, oder auf einer Verpackung eines Medizin- oder Hygieneproduktes, oder auf einem Bauteil zum Herstellen oder Verarbeiten von Lebensmitteln oder auf einem sonstigen Produkt, bei dem eine besondere Hygiene erforderlich ist. Wie eingangs geschildert besteht insbesondere im Bereich der Medizin- und Hygieneprodukte ein Bedarf an antimikrobiellen und gleichzeitig nicht cytotoxischen Produkten. Durch Versehen herkömmlicher Produkte mit einem erfindungsgemäßen Schichtmaterial - beispielsweise in Form einer Beschichtung mit dem Schichtmaterial - kann dieser Bedarf besonders einfach gestillt werden.

Die Erfindung wird nachfolgend anhand bevorzugter Ausführungsformen näher beschrieben. Dabei zeigen
Figur 1: Einen Querschnitt eines antimikrobiellen und nicht zytotoxischen Schichtmaterials,
Figur 2: Einen zeitlichen Verlauf des bakteriellen Bewuchses verschiedener Polyu rethan-Oberflächen

### Beispiel 1: Herstellen eines erfindungsgemäßen Schichtmaterials

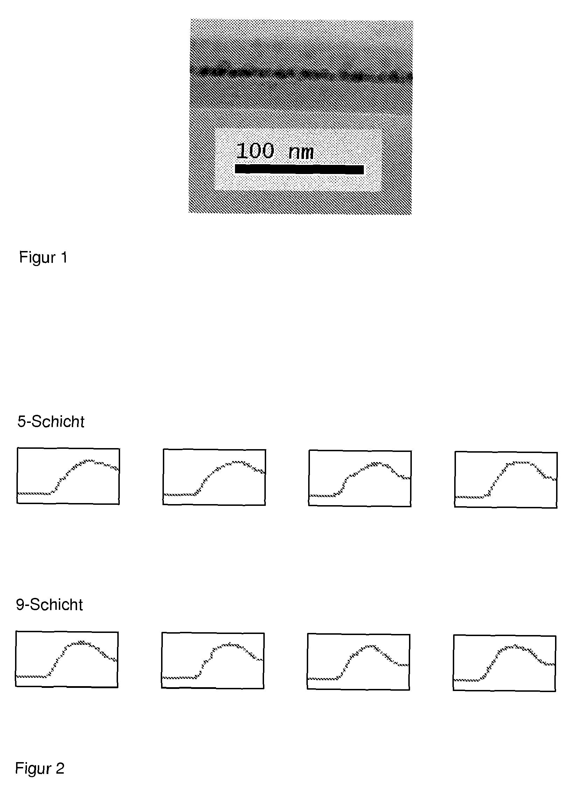

Ein 25x10x10 mm³ großes Polystryrol-Substrat wird im Vakuum bei 1.5 * 10⁻² mbar einer Plasmafeinreinigung unterzogen (O₂-Fluss = 100 sccm, P = 1000 W, t = 10 min). Anschließend wird einer SiO₂-Beschichtung mit Hilfe eines HMDSO/O₂-Plasmas (HMDSO-Fluss = 10 sccm, O₂ = 100 sccm, P = 1000 W) durchgeführt. Nach einer Abscheidedauer von 10 min entsteht auf dem Substrat eine ca. 50 nm dicke Schicht. Auf diese Schicht werden bei einem H₂O-Partialdruck von 5*10⁻³ mbar mit einer HF-Sputterquelle Silberteilchen aufgebracht. Die Sputterzeit beträgt 10 min und die Sputterleistung 1000 W. Die Transportkontrollschicht wird wie schon die erste SiO₂-Schicht wieder hergestellt durch Plasmapolymerisation bei einem Druck von 0.015 mbar, einer Plasmaleistung von 1000 W, einem HMDSO-Fluss von 10 sccm und einem O₂-Fluss von 100 sccm. Nach 10 min Abscheidedauer entsteht eine ca. 50 nm dicke SiO₂-Schicht als Transportkontrollschicht. Die TEM-Aufnahme dieser Beschichtung zeigt Figur 1.

### Beispiel 2: Herstellen eines erfindungsgemäßen Schichtmaterials

Zur Herstellung eines 5-fach bzw. 9-fach-Schicht-Systems auf einem Metallstab mit einer Länge von 20 cm und einem Durchmesser von 5 mm wird zuerst eine Plasmafeinreinigung mit den Parametern aus Beispiel 1 durchgeführt. Anschließend erfolgt die Abscheidung einer SiO₂-Beschichtung und der Silberpartikel, ebenfalls wie in Beispiel 1 beschrieben. Werden diese beiden Beschichtungen einmal wiederholt, so entsteht nach der abschließenden Beschichtung mit der Transportkontrollschicht das 5-fach-Schicht-System (SiO₂/Ag/SiO₂/Ag/SiO₂), nach viermaliger Wiederholung das 7-Schicht-System (SiO₂/Ag/SiO₂/Ag/SiO₂/Ag/SiO₂/Ag/SiO₂). Nach der Präparation wurde an diesen Schichten ein SIMS-Tiefenprofil aufgenommen.

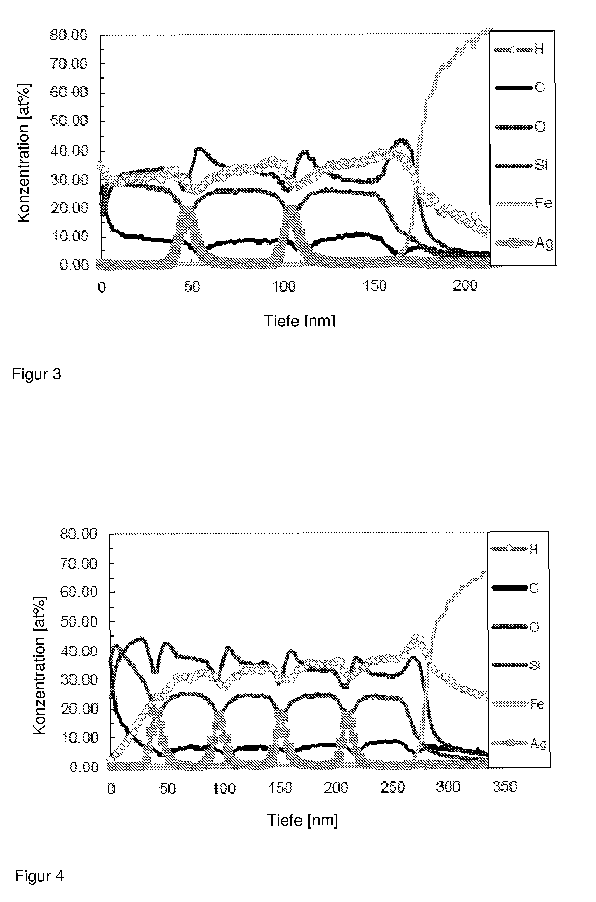

Figur 3 zeigt ein SIMS-Tiefenprofil einer 5-fach-Schicht aus Beispiel 2. Dabei bedeutet "Concentration" die Anteile der jeweils bestimmten Elemente in Atom-%, "Depth" bedeutet den Abstand von der Oberfläche des Metallstabes in nm. Beispiel 5 erläutert die Figur weiter.

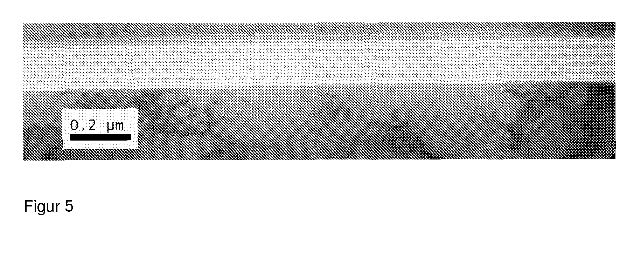

Figur 4 zeigt ein SIMS-Tiefenprofil eines 9-fach-Schicht-Systems aus Beispiel 2. Dabei bedeutet "Concentration" wiederum die Anteile der jeweils bestimmten Elemente in Atom-%, "Depth" bedeutet den Abstand von der Oberfläche des Metallstabes in nm. Beispiel 5 erläutert die Figur weiter. Figur 5 zeigt eine TEM-Aufnahme des 9-fach-Schichtsystems.

Auf die in Beispiel 2 beschriebene Weise können insbesondere Medizinprodukte wie Wundauflagen, Knochennägel und Katheter mit einem erfindungsgemäßen Schichtmaterial beschichtet werden.

### Beispiel 3: Herstellen eines weiteren erfindungsgemäßen Schichtmaterials

Ein mit einem erfindungsgemäßen Schichtmaterial zu versehendes Substrat wird in einem ersten Bearbeitungsschritt mit einem Titandioxidfilm durch Plasmapolymerisation versehen. Als Precursor wird Titantetraisopropyloxid (TTIP) im Gemisch mit Sauerstoff verwendet. Die Polymerisationszeit beträgt fünf Minuten. Es entsteht ein 25 nm dicker, gut haftender TiO₂-Film. Auf diese Schicht werden bei einem H₂O-Partialdruck von 5*10⁻³ mbar mit einer HF-Sputterquelle Silberteilchen aufgebracht. Die Sputterzeit beträgt 10 min und die Sputterleistung 1000 W. In einem dritten Beschichtungsschritt wird eine Plasmapolymerschicht (Transportkontrollschicht) auf die Silberschicht aufgetragen. Die Plasmapolymerisation erfolgt wieder mit TTIP im Gemisch mit Sauerstoff. Das gesamte Schichtsystem besteht somit aus einem TiO₂/Ag/TiO₂-Aufbau. Das Titanoxid lässt sich zusätzlich durch UV-Strahlung aktivieren, d.h. die Oberflächenenergie nimmt temporär stark (über 72 mN/m) zu.

Mit dem erfindungsgemäßen Schichtmaterial sind folgende Materialien besonders gut zu versehen: Metalle, insbesondere Titan und (ggf. medizinischer) Edelstahl, Kunststoffe, insbesondere Polyurethan, und Cellulose, insbesondere Wundauflagen und Cellulosevliese.

### Beispiel 4: Herstellung eines weiteren erfindungsgemäßen Schichtmaterials

Im Beispiel 3 werden die TiO₂-Schichten durch Plasmapolymerisation des titanorganischen Precursors TTIP hergestellt. Alternativ hierzu kann Titanoxid auch durch reaktives Magnetron-Sputtern präpariert werden. Hierzu wird unter einer Ar/O₂-Atmosphäre (O₂-Partialdruck = 7·10⁻⁵ mbar) von einem metallischen Ti-Target bei einer DC-Sputterleistung von 4 kW gesputtert. Die statische Schichtabscheiderate beträgt bei diesem Prozess ca. 30 nm/min.

### Beispiel 5: Untersuchung eines gemäß Beispiel 2 hergestellten erfindungsgemäßen Schichtmaterials

Das 5-fach- (Figur 3) und das 9-fach-Schicht-System (Figur 4) aus Beispiel 2 wurde mit Sekundärionenmassenspektrometrie (SIMS) untersucht. Hierbei wird in die Beschichtung mit einem gerichteten Ar-lonen-Strahl ein Loch geschossen. Das hierbei abgehobene (abgesputterte) Material wird mit SIMS analysiert. Durch einen kontinuierlichen Betrieb der Ar-Ionenquelle und des SIMS wird eine tiefenabhängige Elementanalyse möglich. Als Referenzmaterial wurde SiO₂ gewählt, weil der Sauerstoffanteil in der Beschichtung einen ähnlich hohen Sauerstoffanteil aufweist. Aufgenommen wurden die Elemente Wasserstoff (H), Sauerstoff (O), Silizium (Si), Silber (Ag), Eisen (Fe) und Kohlenstoff (C).

Die Gesamtschichtdicke der 5-fach-Schicht beträgt ca. 150 nm und die der 9-fach-Schicht ca. 250 nm. Die Dicke der Beschichtung wird hierbei von der Oberfläche der Beschichtung bis zu der Tiefe gemessen, wo das Eisensignal ansteigt. Die einzelnen SiO₂-Schichten besitzen jeweils eine Dicke von ca. 50 nm, die jeweils von einer ca. 10 nm dicken Silberschicht getrennt sind. Die Silberkonzentration in den einzelnen Ag-Schichten ist in etwa gleich.

Figur 2 zeigt einen Nachweis der antimikrobiellen Wirkung einer gemäß Beispiel 1 mit einem erfindungsgemäßen Schichtmaterial versehenen Polyurethan-Oberfläche im Vergleich zu einer unbehandelten Polyurethan-Oberfläche. Die antimikrobielle Wirkung wurde wie in der DE 197 58 598 A1 beschrieben mit *Staphylococcus epidermidis* geprüft. Figur 2 zeigt die Entwicklung der optischen Dichte und damit der Bakterienzahl über einen Zeitraum von 48 Stunden. Dargestellt ist jeweils die Entwicklung des bakteriellen Bewuchses der oben beschriebenen 5-fach-Schicht ("5-Layer") und der oben beschriebenen 9-fach-Schicht ("9-Layer"), jeweils in 4 unabhängigen Versuchen.

Auf einer unbehandelten Polyurethan-Oberfläche erfolgt innerhalb kürzester Zeit ein bakterielles Wachstum (nicht gezeigt), während auf den erfindungsgemäßen Schichtmaterialien innerhalb des dargestellten Zeitraums ein deutlich verzögertes bakterielles Wachstum stattfindet. Das erfindungsgemäße Schichtmaterial ist demnach antimikrobiell. Es ist zudem gemäß DIN-ISO10993-5 nicht zytotoxisch (hierzu keine Figur).

## Patentansprüche

1. Antimikrobielles und nicht zytotoxisches Schichtmaterial, umfassend
a) eine Trägerschicht,
b) zumindest eine auf der Trägerschicht aufgebrachte Biozid-Schicht mit einem anorganischen bioziden Wirkstoff, ausgewählt aus der Gruppe bestehend aus Silber, Kupfer und Zink, deren Ionen und deren Metallkomplexe, oder einer Mischung oder Legierung umfassend zwei oder mehr dieser Elemente, und
c) eine auf eine Biozid-Schicht aufgebrachte Transportkontrollschicht, wobei die Transportkontrollschicht eine Gasdurchlässigkeit für Sauerstoff (02) aufweist von 50 bis unter 100 (cm3 bar) / (Tag m2), wobei die Trägerschicht und die Transportkontrollschicht das gleiche Grundmaterial besitzen.

2. Schichtmaterial nach Anspruch 1, **dadurch gekennzeichnet, dass** die Transportkontrollschicht eine Gasdurchlässigkeit für Sauerstoff (02) aufweist von 80 bis unter 100 (cm3 bar) / (Tag m2), vorzugsweise von 90 bis unter 100 (cm3 bar) / (Tag m2).

3. Schichtmaterial nach Anspruch 1 oder 2, wobei der biozide Wirkstoff eine mittlere Korngröße von 5 bis 100 nm hat.

4. Schichtmaterial nach einem der vorherigen Ansprüche, wobei die Biozid-Schicht ferner umfasst: Gold, Platin, Palladium, Iridium, Zinn, Antimon, deren Ionen, deren Metallkomplexe, oder eine Legierung des bioziden Wirkstoffs mit einem oder mehreren dieser Elemente.

5. Schichtmaterial nach einem der vorherigen Ansprüche, wobei die Transportkontrollschicht und die Trägerschicht ein Grundmaterial besitzen, das ausgewählt ist aus der Gruppe bestehend aus
a) einem organischen Grundmaterial, insbesondere einem Plasmapolymer, einem Sol-Gel, einem Lack, und einem silikonisierten Grundmaterial, oder
b) einem anorganischen Grundmaterial, insbesondere SiO₂ und SiC, einem Metalloxid, insbesondere TiO₂ und Al₂O₃, und einem nichtbioziden Metall, insbesondere Titan oder medizinischem Edelstahl.

6. Schichtmaterial nach Anspruch 5, wobei die Transportkontrollschicht einen Silizium-Anteil von 20 bis 60 %, einen Kohlenstoffanteil von 10 bis 30 % und einen Sauerstoffanteil von 30 bis 50 % besitzt.

7. Schichtmaterial nach einem der vorherigen Ansprüche, wobei die Biozid-Schicht eine mittlere Dicke von 5 bis 100 nm hat, und/oder wobei die Transportkontrollschicht eine mittlere Dicke von 5 bis 500 nm hat.

8. Schichtmaterial nach einem der vorherigen Ansprüche, **dadurch gekennzeichnet, dass** die Transportkontrollschicht einen Silizium-Anteil von 20 bis 60 %, einen Kohlenstoffanteil von 10 bis 30 % und einen Sauerstoffanteil von 30 bis 50 % besitzt.

9. Schichtmaterial nach einem der vorherigen Ansprüche, **dadurch gekennzeichnet, dass** das Schichtmaterial mehrere Trägerschichten und Biozidschichten umfasst, so dass zumindest eine Biozidschicht eingebettet ist zwischen zwei Trägerschichten.

10. Verwendung eines Schichtmaterials nach einem der Ansprüche 1 bis 9 zum Herstellen einer antimikrobiellen und nicht zytotoxischen Beschichtung auf einem festen Körper.

11. Verwendung eines Schichtmaterials nach einem der Ansprüche 1 bis 9 zum Herstellen einer antimikrobiellen und nicht zytotoxischen Beschichtung auf einem Medizinprodukt, insbesondere einem Katheter, einer Wundauflage, einer Kontaktlinse, einem Implantat, einem medizinischen Nagel und/oder Schraube, Knochenfixationsnagel, einem medizinischen Instrument, oder auf einem Hygieneprodukt, insbesondere auf einer Binde oder Windel, oder auf einer Verpackung eines Medizin- oder Hygieneproduktes, oder auf einem Bauteil zum Herstellen oder Verarbeiten von Lebensmitteln oder auf einem sonstigen Produkt, bei dem es eine besondere Hygiene erforderlich ist.

12. Verwendung einer Transportkontrollschicht mit einer Gasdurchlässigkeit für Sauerstoff (O₂) im Bereich von 50 bis unter 1000 (cm³ bar) / (Tag m²), vorzugsweise im Bereich von 80 bis unter 100 (cm³ bar) / (Tag m²), besonders bevorzugt von 90 bis unter 100 (cm³ bar) / (Tag m²), zum Herstellen eines Schichtmaterials nach einem der Ansprüche 1 bis 9.

## Claims

1. Antimicrobial and non-cytotoxic coating material, comprising
a) a carrier layer,
b) at least one biocide layer containing an inorganic biocidal agent applied to the carrier layer, selected from the group consisting of silver, copper and zinc, their ions and their metal complexes, or a mixture or alloy comprising two or more of these elements, and
c) a transport control layer applied to a biocide layer, wherein the transport control layer has a gas permeability to oxygen (02) of 50 to less than 100 (cm3 bar)/(day m2), wherein the carrier layer and the transport control layer have the same base material.

2. Coating material according to claim 1, **characterised in that** the transport control layer has a gas permeability to oxygen (02) of 80 to less than 100 (cm3 bar)/(day m2), preferably of 90 to less than 100 (cm3 bar)/(day m2).

3. Coating material according to claim 1 or 2, wherein the biocidal agent has an average particle size of 5 to 100 nm.

4. Coating material according to one of the preceding claims, wherein the biocide layer further comprises: gold, platinum, palladium, iridium, tin, antimony, their ions, their metal complexes, or an alloy of the biocidal agent with one or more of these elements.

5. Coating material according to one of the preceding claims, wherein the transport control layer and the carrier layer have a base material selected from the group consisting of
a) an organic base material, in particular a plasma polymer, a solgel, a lacquer, and a siliconised base material, or
b) an inorganic base material, in particular SiO₂ and SiC, a metal oxide, in particular TiO₂ and Al₂O₃, and a non-biocidal metal, in particular titanium or medical stainless steel.

6. Coating material according to claim 5, wherein the transport control layer has a silicon content of 20 to 60%, a carbon content of 10 to 30% and an oxygen content of 30 to 50%.

7. Coating material according to one of the preceding claims, wherein the biocide layer has an average thickness of 5 to 100 nm, and/or wherein the transport control layer has an average thickness of 5 to 500 nm.

8. Coating material according to one of the preceding claims, **characterised in that** the transport control layer has a silicon content of 20 to 60%, a carbon content of 10 to 30% and an oxygen content of 30 to 50%.

9. Coating material according to one of the preceding claims, **characterised in that** the coating material comprises a plurality of carrier layers and biocide layers, so that at least one biocide layer is embedded between two carrier layers.

10. Use of a coating material according to one of claims 1 to 9 for producing an antimicrobial and non-cytotoxic coating on a solid body.

11. Use of a coating material according to one of claims 1 to 9 for producing an antimicrobial and non-cytotoxic coating on a medical product, in particular a catheter, a wound covering, a contact lens, an implant, a medical nail and/or screw, bone fixation nail, a medical instrument, or on a sanitary product, in particular on a sanitary towel or nappy, or on a packaging of a medical or sanitary product, or on a component for producing or processing foodstuffs or on another product for which particular hygiene is required.

12. Use of a transport control layer with a gas permeability to oxygen (O₂) in the range from 50 to less than 1000 (cm³ bar)/(day m²), preferably in the range from 80 to less than 100 (cm³ bar)/(day m²), particularly preferably from 90 to less than 100 (cm³ bar)/(day m²), for producing a coating material according to one of claims 1 to 9.

## Revendications

1. Matériau stratifié antimicrobien et non cytotoxique, comprenant
a) une couche support,
b) au moins une couche biocide appliquée sur la couche support avec une substance biocide inorganique, choisie dans le groupe composé d'argent, de cuivre et de zinc, leurs ions et leurs complexes métalliques ou dans un mélange ou alliage comprenant deux ou plusieurs de ces éléments, et
c) une couche de contrôle du transport appliquée sur une couche biocide, la couche de contrôle du transport présentant une perméabilité au gaz pour l'oxygène (O₂) de 50 à moins de 100 (cm³ bar) / (jour m²) et la couche support et couche de contrôle du transport possédant le même matériau de base.

2. Matériau stratifié selon la revendication 1, **caractérisé en ce que** la couche de contrôle du transport présente une perméabilité au gaz pour l'oxygène (O₂) de 80 à moins de 100 (cm³ bar) / (jour m²), de préférence de 90 à moins de 100 (cm³ bar) / (jour m²).

3. Matériau stratifié selon la revendication 1 ou 2, **caractérisé en ce que** la substance biocide a une granulométrie moyenne de 5 à 100 nm.

4. Matériau stratifié selon l'une des revendications précédentes, dans lequel la couche biocide comprend en outre : de l'or, du platine, du palladium, de l'iridium, de l'étain, de l'antimoine, leurs ions, leurs complexes métalliques ou un alliage de la substance biocide composé d'un ou de plusieurs de ces éléments.

5. Matériau stratifié selon l'une des revendications précédentes, dans lequel la couche de contrôle du transport et la couche support comprennent un matériau de base choisi dans le groupe composé de
a) un matériau de base organique, notamment un polymère à plasma, un sol-gel, un vernis et un matériau de base siliconé, ou
b) un matériau de base inorganique, notamment SiO₂ et SiC, un oxyde métallique, notamment TiO₂ et Al₂O₃ et un métal non biocide, notamment du titane ou un acier inoxydable médical.

6. Matériau stratifié selon la revendication 5, dans lequel la couche de contrôle du transport a une teneur en silicium de 20 à 60 %, une teneur en carbone de 10 à 30 % et une teneur en oxygène de 30 à 50 %.

7. Matériau stratifié selon l'une des revendications précédentes, dans lequel la couche biocide a une épaisseur moyenne de 5 à 100 nm et/ou dans lequel la couche de contrôle du transport a une épaisseur moyenne de 5 à 500 nm.

8. Matériau stratifié selon l'une des revendications précédentes, **caractérisé en ce que** la couche de contrôle du transport a une teneur en silicium de 20 à 60 %, une teneur en carbone de 10 à 30 % et une teneur en oxygène de 30 à 50%.

9. Matériau stratifié selon l'une des revendications précédentes, **caractérisé en ce qu'**il comprend plusieurs couches support et plusieurs couches biocides, de sorte qu'au moins une couche biocide est placée entre deux couches support.

10. Utilisation d'un matériau stratifié selon l'une des revendications 1 à 9 pour la production d'un revêtement antimicrobien et non cytotoxique sur un corps solide.

11. Utilisation d'un matériau stratifié selon l'une des revendications 1 à 9 pour la production d'un revêtement antimicrobien et non cytotoxique sur un dispositif médical, notamment un cathéter, un pansement, une lentille de contact, un implant, un clou et/ou une vis médicaux, un clou de fixation osseuse, un instrument médical ou sur un produit d'hygiène, notamment sur une serviette hygiénique ou une couche, ou sur l'emballage d'un produit médical ou d'hygiène, ou sur un composant pour la production ou la transformation de produits alimentaires ou sur un produit quelconque qui nécessite une hygiène particulière.

12. Utilisation d'une couche de contrôle du transport avec une perméabilité au gaz pour l'oxygène (O₂) d'environ 50 jusqu'à moins de 1 000 (cm³ bar)/(jour m²), de préférence dans l'intervalle de 80 à moins de 100 (cm³ bar)/(jour m²) et de façon particulièrement préférée dans l'intervalle de 90 à moins de 100 (cm³ bar) / (jour m²) pour la production d'un matériau stratifié selon l'une des revendications 1 à 9.
